# EUROPEAN PATENT APPLICATION

(11) **EP 4 174 169 A1**
(43) Date of publication of application: **03.05.2023**
(21) Application number: 20942249.2
(22) Date of filing: 25.06.2020
(51) Int. Cl.: C12N 5/071, C12M 3/00, C12M 1/32, C12M 1/00

(54) **STANDARD ORGANOID PRODUCTION METHOD**

(71) Applicant: Next & Bio Inc., Seoul 08826 (KR)
(72) Inventor: CHUNG, Seok, Seoul 04024 (KR); YANG, Ji Hoon, Seoul 04413 (KR); JUNG, Yong Hun, Seoul 02860 (KR); CHOI, Dong Hee, Seoul 02578 (KR)
(74) Representative: Kador & Partner Part mbB
(86) International application number: PCT/KR2020/008274
(87) International publication number: WO 2021/261622

(57) **Abstract**

The present invention provides a method of producing a standard-type organoid.

## Description

### [Technical Field]

The present invention relates to a method of producing a standard-type organoid. More specifically, the present invention relates to a method of producing an organoid having a uniform size.

### [Background Art]

An organoid is also called "an organ analog" or "organ-like" and is an organ-specific cell aggregate produced by re-aggregating and recombining cells separated from stem cells or organ-originating cells by a 3D culture method. The organoid includes specific cells of a model organ, reproduces the specific functions of the organ, and can be spatially organized in a form similar to the actual organ. It has been reported that a patient-derived tumor organoid represents the characteristics of the patient's cancer cells and tissues as they are, and can reproduce the genetic variation characteristics of the patient's cancer tissues.

Organoids can be used in the fields of cell therapy, biotissue engineering, new drug development, toxicology and precision medicine. A large amount of comparable quantitative organoids and analytical methods thereof are required to enhance the utilization of organoids. However, there has been no method of quantitatively culturing organoids to date. The reason is that after Matrigel, which is the most important of the elements that grow organoids, is solidified in a dome-shape on the bottom, the organoids are grown therein, such that the organoids grow separately.

Further, since the organoids so grown in Matrigel can be grown while overlapping in a 3D support, the limits are clear.

In addition, recently, high-throughput screening techniques have been developed, which combine more stable and physiological patient-derived organoids for use in early drug discovery programs and toxicity screening.

Korean Patent No. 10-1756901 (Patent Document 1) discloses a cell culture chip capable of culturing 3D tissue cells. In the cell culture chip of Patent Document 1, a first culture part, a second culture part, and the third culture part are formed in each layer, and the degree of cell growth progress can be confirmed in each layer. However, the cell culture chip of Patent Document 1 has a problem in that organoids cannot be obtained in high yield.

Further, there is a case where the pipetting work of replacing a culture solution during cell culture is performed, and in the case of a Coming spheroid microplate capable of 3D cell culture, spheroids or organoids in cell culture are affected, so that there is a problem which is not good for the cell culture environment because there is a case where the spheroids or organoids are sucked up or the positions thereof are changed during the pipetting work.

Thus, the present inventors have conducted continuous studies on a standard-type organoid having a uniform size while using no extracellular matrix-based hydrogel (for example, Matrigel) or minimizing the use of the extracellular matrix-based hydrogel, thereby completing the present invention.

### [Related Art Documents]

### [Patent Document]

1. Korean Patent No. 10-1756901

### [Disclosure]

### [Technical Problem]

An object of the present invention is to provide a method of producing a standard-type organoid.

Another object of the present invention is to provide a standard-type organoid having a uniform size and similar functionality of each organoid, which is produced by the above method.

However, a technical problem to be achieved by the present invention is not limited to the aforementioned problems, and other problems that are not mentioned may be clearly understood by those skilled in the art from the following description.

### [Technical Solution]

To achieve the objects, the present invention is
a method of producing an organoid, the method including:
forming an organoid by culturing cells in a 3D cell culture plate,
wherein in the forming of the organoid, the cell culture plate includes 0 to 2 vol% of an extracellular matrix-based hydrogel, and
wherein the 3D cell culture plate includes:
   a well plate including a plurality of main wells and a plurality of sub wells formed at lower portions of the main wells to be injected with a cell culture solution and including recessed parts on a bottom surface thereof; and a connector for large-capacity and high-speed high content screening (HCS), which supports the well plate, and
   the connector for high content screening (HCS) includes a base equipped with a fixing means so as to be attached to and detached from a lower end of the well plate and a cover positioned on an upper portion of the well plate to be coupled to the base, the main well has a step formed so as to be tapered at a predetermined site, and the step has an inclination angle (θ) ranging from 10 to 60° with respect to a wall of the main well.

The cells may be normal cells or cancer cells.

The cells may be single cells.

The cells may be obtained by being isolated from a normal tissue, a cancer tissue, or an already produced organoid. Since the method of making a tissue cells and separating the cells into single cells is a known technique, a specific description thereof will be omitted.

The cell culture period is preferably 1 to 14 days.

The extracellular matrix-based hydrogel may be Matrigel (product name).

A size of the organoids may be 300 to 500 µm in diameter.

As used herein, the term "standard-type organoid" refers to an organoid having a uniform size of 300 to 500 µm in diameter.

The size of the organoids produced in the present invention ranges from 300 to 500 µm, which is a size particularly optimized for cancer diseases.

As will be described in detail below, when the 3D cell culture plate of the present invention is used, a standard-type organoid may be mass-produced.

The sub well of the 3D cell culture plate may have an inclined surface formed so as to taper toward the recessed part, the sub wells may have an upper end diameter ranging from 3.0 to 4.5 mm, the recessed parts may have an upper end diameter ranging from 0.45 to 1.5 mm, an inclined surface (θ2) between the sub well and the recessed part may range from 40 to 50°, and a length ratio of the diameter of the sub wells to the diameter of the recessed parts may range from 1:0.1 to 0.5.

The main wells of the 3D cell culture plate may have an individual volume ranging from 100 to 300 µl, the recessed parts may have an individual volume ranging from 20 to 50 µl, and an individual volume ratio of the main well to the recessed part may be 1 : 0.1 to 0.5 on average.

The main well includes a space part between the step and the sub well, the space part may have a height (aₕ) ranging from 2.0 to 3.0 mm on average, the sub well may have a height (bₕ) from 1.0 to 2.0 mm on average, and a height ratio (aₕ:bₕ) of the space part to the sub well may range from 1:0.3 to 1.

The cells may be seeded on the cell culture plate at 100 to 300 cells/well.

Hereinafter, the present invention will be described in detail.

Since the present invention may be modified in various forms and include various exemplary embodiments, specific exemplary embodiments will be illustrated in the drawings and described in detail in the Detailed Description.

However, the description is not intended to limit the present invention to the specific embodiments, and it is to be understood that all the changes, equivalents and substitutions included in the idea and technical scope of the present invention are included in the present invention. When it is determined that the detailed description of the related publicly known art in describing the present invention may obscure the gist of the present invention, the detailed description thereof will be omitted.

The terms used in the present application are used only to describe specific embodiments, and are not intended to limit the present invention. Singular expressions include plural expressions unless the context clearly indicates otherwise.

In the present invention, the term "include" or "have" is intended to indicate the presence of the characteristic, number, step, operation, constituent element, part or any combination thereof described in the specification, and should be understood that the presence or addition possibility of one or more other characteristics or numbers, steps, operations, constituent elements, parts or any combination thereof is not pre-excluded.

In general, when organoids are cultured, a hydrogel is used to provide the role of an extracellular matrix. For example, after Matrigel is solidified in a dome-shape on the bottom of a cell culture plate, organoids are grown therein, and organoids grow in different sizes and shapes and the functions thereof develop differently for this reason, such that there is a problem in that it is difficult to standardize the organoids.

The present invention produces organoids using a 3D cell culture plate which does not include, or includes a minimal amount of extracellular matrix-based hydrogel. A specific description on the 3D cell culture plate of the present invention is as follows.

In an exemplary embodiment, the present invention uses a 3D cell culture plate including:
a well plate including a plurality of main wells and a plurality of sub wells formed at lower portions of the main wells to be injected with a cell culture solution and including recessed parts on a bottom surface thereof; and
a connector for large-capacity and high-speed high content screening (HCS), which supports the well plate,
wherein the connector for high content screening (HCS) includes a base equipped with a fixing means so as to be attached to and detached from a lower end of the well plate and a cover positioned on an upper portion of the well plate to be coupled to the base,
the main well has a step formed so as to be tapered at a predetermined site, and the step has an inclination angle (θ) ranging from 10 to 60° with respect to a wall of the main well.

A 96-well plate in the related art has a problem in that it takes a lot of time and costs because experiments and analyses should be performed several times or more in order to evaluate the efficacy of a drug in high yield. Furthermore, there is a case where the pipetting work of replacing a culture solution during cell culture is often performed, and in the case of a Coming spheroid microplate in the related art, spheroids or organoids in cell culture are affected, so that there was a problem which is not good for the cell culture environment because there is a case where spheroids or organoids are sucked up or the positions thereof are changed during the pipetting work.

Therefore, the present invention has been made in an effort to solve the above-described problems, and provides a cell culture plate capable of manufacturing spheroids/organoids in high yield by including a plurality of sub wells in a plurality of main wells in a well plate, and capable of uniformly capturing images in the well plate by including a connector for large-capacity high-speed high content screening (HCS), which supports the well plate to reduce a tolerance when a large-capacity and high-speed image is captured. Furthermore, the present invention provides a cell culture plate capable of minimizing the effects of the pipetting work during replacement of a medium on cells to be cultured by the step of the main well.

Hereinafter, preferred exemplary embodiments of the present invention will be described in detail with reference to the accompanying drawings. Prior to the description, terms or words used in the specification and the claims should not be interpreted as being limited to a general or dictionary meaning and should be interpreted as a meaning and a concept which conform to the technical spirit of the present invention based on a principle that an inventor can appropriately define a concept of a term in order to describe his/her own invention by the best method.

Accordingly, since the exemplary embodiments described in the present specification and the configurations illustrated in the drawings are only the most preferred exemplary embodiments of the present invention and do not represent all of the technical spirit of the present invention, it is to be understood that various equivalents and modified examples, which may replace the exemplary embodiments and the configurations, are possible at the time of filing the present application.

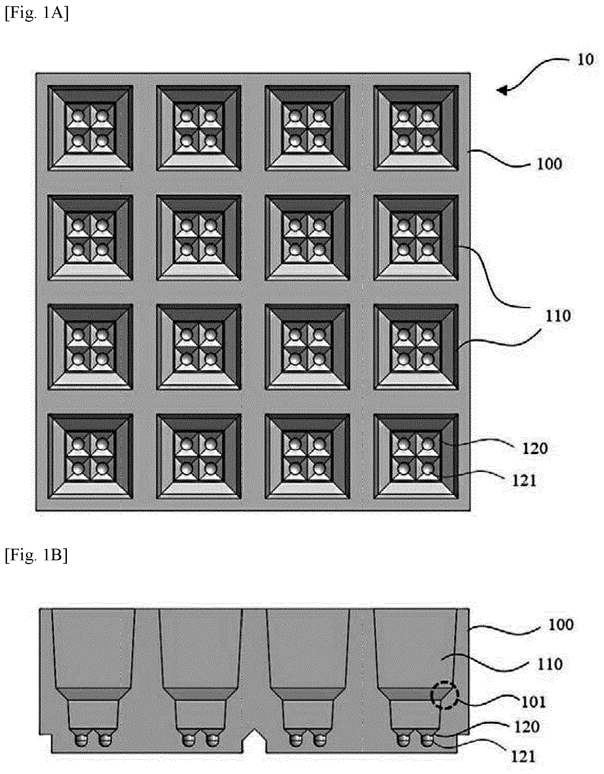

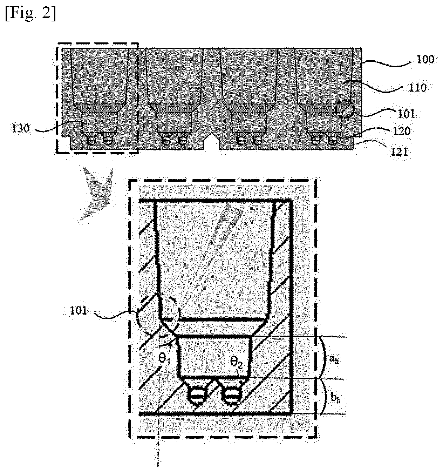

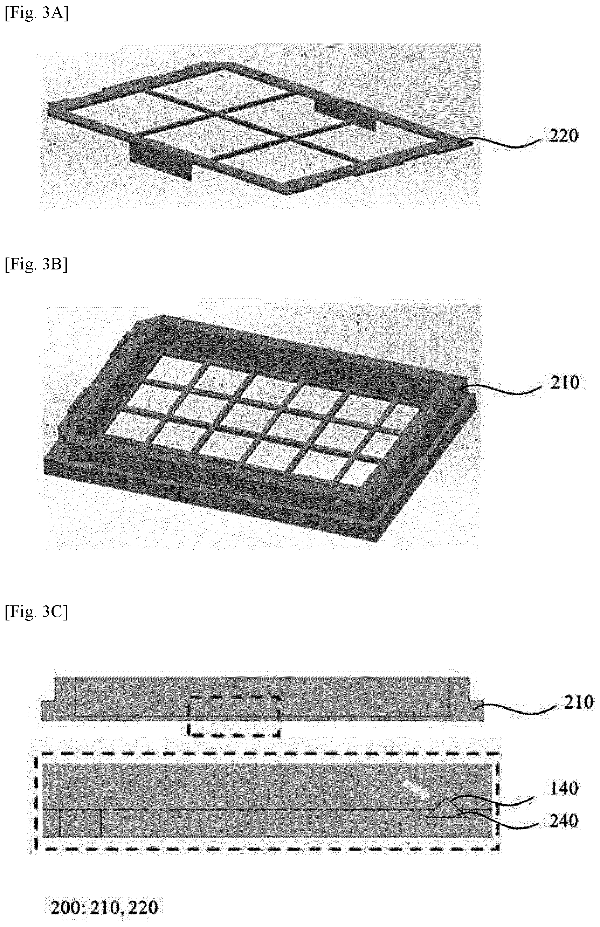

FIG 1A is a front view of a cell culture plate according to an exemplary embodiment of the present invention , FIG 1B is a cross-sectional view of the cell culture plate according to an exemplary embodiment of the present invention, FIG 2 is a view illustrating a main well formed in the cell culture plate according to an exemplary embodiment of the present invention, and FIG 3 is a view illustrating a well plate, a base and a cover of the cell culture plate according to an exemplary embodiment of the present invention ((A) a cover, (B) a base, and (C) a fixing means of a microplate and a base).

Hereinafter, a cell culture plate according to an exemplary embodiment of the present invention will be described in detail with reference to FIGS. 1 to 3.

As illustrated in FIGS. 1 to 3, a cell culture plate 10 according to an exemplary embodiment of the present invention includes a well plate 100 including a plurality of main wells 110 and a plurality of sub wells formed at lower portions of the main wells 10 to be injected with a cell culture solution and including recessed parts 121 on a bottom surface thereof; and a connector 200 for large-capacity and high-speed high content screening (HCS), which supports the well plate 100.

First, the well plate 100 according to an exemplary embodiment of the present invention will be described in detail.

The well plate 100 is made into a plate shape that is plastic injection-molded through a mold. In order to manufacture a mold for plastic injection as described above, the main well 110 has a repeating pattern as a well structure such that the unit cost of production can be reduced and the size can be easily increased using fine machining. Therefore, cells are easily mass-produced, and the cells can be transformed into various sizes according to the user's requirements and used.

A plurality of the main wells 110 is formed in the well plate 100, and each main well 110 includes a step 101. The step 101 is formed at a predetermined site of the main well 110, and more specifically, the step 101 may be formed at a position which is 1/3 to 1/2 of a total length of the main well 110, and the step 101 may be formed at a position which is 1/3 to 1/2 of the total length from the lower end of the main well 110.

In the related art, there is a case where the pipetting work of replacing a culture solution during cell culture is performed, and in this case, spheroids or organoids in cell culture are affected, so that there is a problem which is not good for the cell culture because there is a case where the spheroids or organoids are sucked up or the positions thereof are changed during the pipetting work, but the step 101 is provided to prevent this problem.

The step 101 may be a space to which a pipette is applied, and specifically, may have an inclination angle (θ) ranging from 10 to 60° with respect to a wall of the main well 110. Alternatively, the step 101 may have an inclination angle ranging from 20 to 50°, preferably ranging from 30 to 45°. When the inclination angle of the step 101 is less than 10°, the inclination angle within the main well 110 is so small that the space to which a pipette can be applied is not sufficient, and as a result, when the culture solution in the main well 110 is sucked up, the pipette may slide inside the sub well 120, causing spheroids or organoids to be sucked up, or the positions thereof, and the like to be changed. Furthermore, when the inclination angle (θ) exceeds 60°, a space to which a pipette can be applied is provided, but the inclination angle of the step 101 is so large that it may be difficult to sufficiently suck up the culture solution, and when cells are seeded in the sub well 120, a problem in that cells are seeded on the step 101 without entering all the sub wells 120 may occur. Therefore, it is desirable to have an inclination angle in the above-described range.

Meanwhile, the main well according to an exemplary embodiment of the present invention may include a space part 130 between the step 101 and a sub well 120 to be described below. Specifically, the space part 130 is a space into which a culture solution is injected, and is a space in which cells inside the sub well 120 can share the same culture solution.

More specifically, the space part 130 may have a height (aₕ) ranging from 2.0 to 3.0 mm on average, or ranging from 2.2 to 2.8 mm, or ranging from 2.3 to 2.7 mm on average. Furthermore, the sub well 120 may have a height (bₕ) ranging from 1.0 to 2.0 mm on average, or ranging from 1.2 to 1.8 mm on average.

For example, the space part 130 may have a height (aₕ) of 2.5 mm on average, and the sub well may have a height (bₕ) of 1.5 mm on average.

In this case, a height ratio (aₕ:bₕ) of the space part to the sub well 120 may range from 1:0.3 to 1, and more specifically, a height ratio (aₕ:bₕ) of the space part to the sub well 120 may be 1:0.4 to 0.9 or 1:0.5 to 0.8. When a ratio of the height of the space part to the height of the sub well 120 is less than 1:0.3, the cells in culture may escape from the inside even with a small force during the exchange of the media of the sub well 120, and when a ratio of the height of the space part to the height of the sub well 120 exceeds 1:1, the culture solution required for the cells is not sufficiently converted, so that cell death may be induced. Therefore, it is preferred that the space part 130 and the sub well 120 have the above-described height range and height ratio.

Next, the sub wells 120 are formed at lower portion of each of the main wells 110 and include recessed parts 121 on a bottom surface thereof. As a particular aspect, the sub well 120 may include a plurality of recessed parts at lower portions of the main well 110.

The sub wells 120 included at the lower portion of the main well 110 have the same size and shape, thereby enabling spheroids and organoids to be produced under uniform conditions.

The sub well 120 may have an inclined surface formed so as to taper toward the recessed part 121. Specifically, the horizontal area of the upper portion of the sub well 120 may become smaller as it descends in the vertical direction. For example, the upper portion of the sub well 120 may be formed in an inverted pyramid shape. In the illustrated exemplary embodiment, the upper portion of the sub well 120 may be formed in a shape such as a pyramid shape or a funnel shape in which the horizontal area of the upper portion of the sub well 120 becomes smaller as it descends in the vertical direction.

In particular, the cell culture plate may produce a large amount of spheroids or organoids under uniform conditions by including a plurality of the sub wells 120 so as to have the same size and shape.

As a particular aspect, one main well 110 can include 4 to 25 sub wells 120 of the same size, and the entire microplate 100 may include 96 to 1,728 sub wells 120. Accordingly, the size can be controlled in the same precise manner.

Furthermore, the sub well 120 includes a recessed part 121, and a space is formed in the lower portion of the recessed part such that 3D spheroids or organoids can be cultured in the recessed part 121. Specifically, the recessed part 121 may be in the form of the letter 'U', 'V', or ' 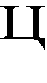 ', and for example, the recessed part 121 may be in the form of the letter 'U'.

The sub well 120 may have an upper end diameter ranging from 3.0 to 4.5 mm, or ranging from 3.5 to 4.3 mm, or 4 mm on average. Furthermore, the recessed part 121 may have an upper end diameter of 0.45 to 1.5 mm, or 0.5 to 1.0 mm or 0.5 mm on average.

Furthermore, a length ratio of the diameter of the sub well 120 to the diameter of the recessed part 121 may range from 1 :0.1 to 0.5, and preferably, a length ratio of the diameter of the sub well 120 to the diameter of the recessed part 121 may be 1:0.12.

When the upper end diameter of the recessed part 121 is less than 0.1 compared to the upper end diameter 1 of the sub well 120, a cell culture space of the recessed part 121 cannot be sufficiently provided, which may cause a problem in that cells escape even with a small force during the replacement of the culture solution, and when the upper end diameter of the recessed part 121 is exceeds 0.5 compared to the upper end diameter 1 of the sub well 120, a sufficient culture solution required for cells cannot be replaced, which may cause a problem in that it is difficult to stably culture cells.

Meanwhile, an inclination surface between the sub well 120 and the recessed part 121 may have an inclination angle (θ₂) of 40 to 50°, 42 to 48°, 43 to 47°, or an inclination angle (θ₂) of 45° on average, with respect to a wall of the main well.

The above-described sub well 120 has an advantage in that cells can be cultured at 100 to 1000 cells/well or less, and the spheroid size can be stably controlled.

Further, the main well 110 according to an exemplary embodiment of the present invention has an individual volume ranging from 100 to 300 µl, the recessed part 121 has an individual volume ranging from 20 to 50 µl, and an individual volume ratio of the main well 110 to the recessed part 121 is characterized by being 1:0.07 to 0.5 on average. Preferably, the main well according to an exemplary embodiment has an individual volume ranging from 250 to 300 µl, the recessed part has an individual volume ranging from 25 to 35 µl, and an individual volume ratio of the main well 110 to the recessed part 121 may be 1:0.11 on average.

Specifically, when the main well 110 has an individual volume less than 100 µl, a problem in that a sufficient culture solution cannot be accommodated during cell culture may occur, and when the individual volume exceeds 300 µl, culture efficiency may be reduced.

Furthermore, the recessed part 121 is a space in which cells are substantially cultured, and when the volume is less than 20 µl, the cell culture space is not sufficient, which may cause a problem in that cells escape, and when the volume exceeds 50 µl, a problem in that it is difficult to stably culture cells and the like may occur. Therefore, it is preferred that the main well 110 and the recessed part 121 have volumes in the above-described ranges.

Due to the above-mentioned configuration of the cell culture plate of the present invention, cells are maintained in the form of a spheroid without including a hydrogel, that is, without coating the cell culture plate with a hydrogel, reprogramming into induced pluripotent stem cells occurs at high efficiency, and the form and function thereof are continuously maintained even after the reprogramming.

The cell culture plate 10 according to an exemplary embodiment of the present invention includes a connector 200 for large-capacity and high-speed high content screening (HCS), which supports the well plate 100. Herein, the connector 200 for large-capacity and high-speed high content screening (HCS) refers to a connector 200 which is attached to a high content screening (HCS) system, and specifically, the connector may refer to a base 210 and a cover 220 in the present invention.

More specifically, the connector for large-capacity and high-speed high content screening (HCS) includes the base 210 equipped with fixing means 140 and 240 so as to be attached to and detached from a lower end of the well plate 100 and a cover 220 positioned on an upper portion of the well plate 100 to be coupled to the base 210. Moreover, the upper end of the base 210 and the lower end of the well plate 100 are characterized by including fixing means 140 and 240 that can be fixed so as to be attached to and detached from each other.

In this case, the base includes a convex part 240 for supporting the well plate 100, and the well plate 100 may include a concave part 140 facing the convex part 240 of the base 210. The well plate 100 may be fixed by the fixing means to uniformly capture images during screening.

The base may be formed of a polyethylene, polypropylene, polystyrene, polyethylene terephthalate, polyamide, polyester, polyvinyl chloride, polyurethane, polycarbonate, polyvinylidene chloride, polytetrafluoroethylene, polyether ether ketone or polyetherimide material, but is not limited thereto.

The well plates may be formed of a polydimethylsilicone, high-fat modified silicone, methyl chlorophenyl silicone, alkyl-modified silicone, methylphenylsilicone, silicone polyester, or amino-modified silicone material, but is not limited thereto.

### [Advantageous Effects]

The method of producing an organoid of the present invention is economical because the use of Matrigel can be minimized. Further, the method of producing an organoid according to the present invention provides an effect of mass-producing a standard-type organoid.

Through the present invention, when large-scale drug screening is performed, organoids having a uniform size, comparable to each other, are formed unlike the conventional organoids, so that the effect of the drug and quantitative analysis become possible. This makes it possible to select a drug suitable for the specificity of each variant gene, and more effective drug treatment can be achieved.

### [Description of Drawings]

FIG 1A is a front view of a cell culture plate according to an exemplary embodiment of the present invention, and FIG 1B is a cross-sectional view of the cell culture plate according to an exemplary embodiment of the present invention.
FIG 2 is a view illustrating, in detail, a main well formed in the cell culture plate according to an exemplary embodiment of the present invention.
FIG 3 is a view illustrating a well plate, a base, and a cover of the cell culture plate according to an exemplary embodiment of the present invention ((A) a cover, (B) a base, and (C) a fixing means of a microplate and a base).
FIG 4 is a view illustrating the high-speed mass imaging results of Example 1 and Comparative Example 1 ((A) Example 1, (B) Comparative Example 1).
FIG 5 illustrates the results of culturing organoids in which Matrigel is included at 2 vol% according to an exemplary embodiment of the present invention and in which Matrigel is not used.
FIG 6 illustrates the results of immunofluorescence staining of organoids including Matrigel at 2 vol% according to an exemplary embodiment of the present invention and in which Matrigel is not used.
FIG 7A is a set of photographs illustrating the high-speed mass imaging results of Example 1, and FIG 7B is a graph illustrating the area of the organoids cultured in Example 1.
FIG 8A is a set of photographs illustrating the high-speed mass imaging results of Comparative Example 1, and FIG 7B is a graph illustrating the area of the organoids cultured in Comparative Example 1.
FIG 9 illustrates imaging results (left) and an organoid size distribution (right) when colorectal cancer cells according to an exemplary embodiment of the present invention are cultured for 14 days.
FIG 10 illustrates organoid stained images (left) and organoid survival rates (right) obtained by culturing colorectal cancer cells according to an exemplary embodiment of the present invention for 14 days.
FIGS. 11 and 12 are a set of photographs and a graph illustrating the high-speed mass imaging results of Examples 1 to 3, respectively ((A) Example 1, (B) Example 2, and (C) Example 3).

### [Modes of the Invention]

Since the present invention may be modified into various forms and include various exemplary embodiments, specific exemplary embodiments will be illustrated in the drawings and described in detail in the Detailed Description. However, the description is not intended to limit the present invention to the specific exemplary embodiments, and it is to be understood that all the changes, equivalents, and substitutions belonging to the spirit and technical scope of the present invention are included in the present invention. When it is determined that the detailed description of the related publicly known art in describing the present invention may obscure the gist of the present invention, the detailed description thereof will be omitted.

### [Examples]

### <Experimental methods>

### 1. Production of organoid

After an existing colorectal cancer organoid was put into a 15 ml tube from a plate by pipetting and the tube was centrifuged at 2000 rpm for 3 minutes, the supernatant was removed, and then a PBS washing process was performed once, and the tube was again centrifuged at 2000 rpm for 3 minutes. Then, the cells were treated with Accutase for 7 minutes and completely separated into single cells. These single cells were seeded in sub wells of a cell culture plate at about 100 cells/well, and an organoid was produced by culturing the single cells for a total of 14 days. In this case, the culture solution is a DMEM/F12-based culture solution, and B27, N2, GlutaMAX, penicillin streptomycin, nicotinamide, N-acetyl, gastrin, A-83-01, EGF, noggin, R-spondin1, and WNT3A are included in the corresponding culture solution, and organoids were produced under culture conditions in which Matrigel was not contained or 2 vol% of Matrigel was contained.

### 2: Measurement of size and number of organoids

The ImageJ program was used for the size analysis of the organoids. Specifically, by selecting a region of interest in a phase image and applying a threshold in the ImageJ program, a part, which was not needed, was overwritten and a part, which was not properly drawn, was filled with black. An area to which the threshold filled with black was applied was calculated using an outer periphery.

### 3: Immunofluorescence staining method

LGR5, which is an organoid stem cell, was stained and confirmed through immunofluorescence staining. First, a standard-type organoid according to the present invention was stored in a 4% paraformaldehyde solution at room temperature for 1 hour, and then stained with PBS. Then, after the standard-type organoid was refrigerated in 15% sucrose for one day and in 30% sucrose for one day, a cryo-block was manufactured using liquid nitrogen. Using the manufactured cryo-block, the block was cut to a thickness of 10 µm, and the cut cross-section was attached to a slide glass. The slide glass was treated with 0.1% TritonX for 10 minutes, and then washed twice with PBS. After the slide glass was stored in 3% BSA at room temperature for 1 hour, the slide glass was washed twice with PBS, and then an LGR5 primary antibody was maintained at room temperature for 2 hours. After washing with PBS, a secondary antibody was treated at room temperature for 2 hours, a mounting solution was added thereto, and measurement was performed under a fluorescence microscope.

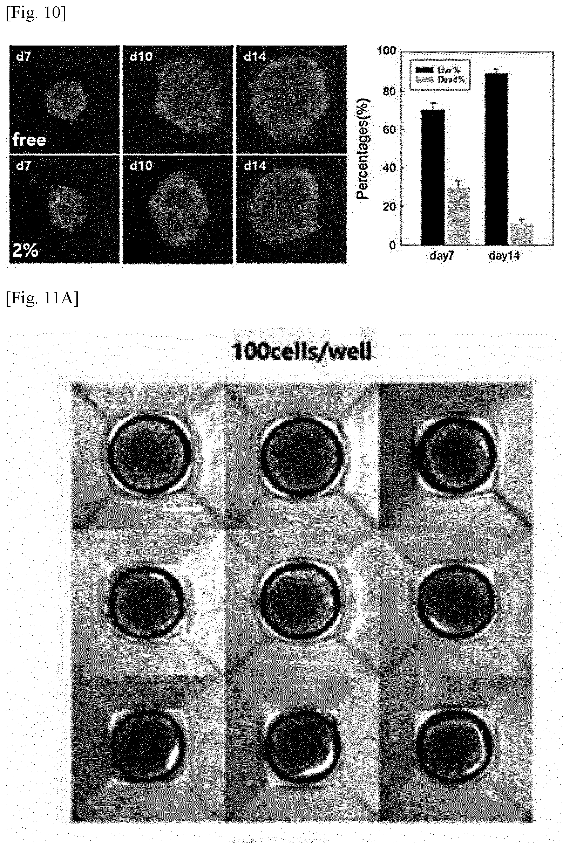

In the case of FIG 10, the cultured standard-type organoid is taken out, and Live/Dead fluorescence staining is performed. In the case of fluorescent staining, 1 mM calcein and 2 mM EtdH-1 are respectively stored at 2 µl per 1 ml and 1 µl per 1 ml in an incubator for 30 to 60 minutes, and then measurement is performed under a fluorescence microscope.

### <Examples>

### Example 1.

Organoids were produced by the method described in Experimental Method 1. Organoids were produced under a condition in which 2 vol% of Matrigel was contained in the culture solution (Example 1-1) and under a condition in which Matrigel was not contained (Example 1-2).

### Example 2,

Cells were cultured in the same manner as in Example 1-1, except that the cells were seeded in sub wells at about 200 cells/well.

### Example 3,

Cells were cultured in the same manner as in Example 1-1, except that the cells were seeded in sub wells at about 300 cells/well.

### Comparative Example 1.

Organoids were cultured in Matrigel, which is a method widely used in the related art, and high-speed mass imaging was performed. However, in the comparative example, a 96-well plate, which is a typically used cell culture plate, was used, and an organoid was produced by seeding cells in Matrigel.

### <Experimental Examples>

### Experimental Example 1. Analysis of organoid images

The cells cultured in Example 1-1 and Comparative Example 1 were photographed, and the sizes of cell spheres were compared. Spheroids were subjected to imaging by an automated plate device, and in this case, the device was allowed to perform imaging by automatically focusing. Image size analysis was performed using a macro program of the ImageJ program.

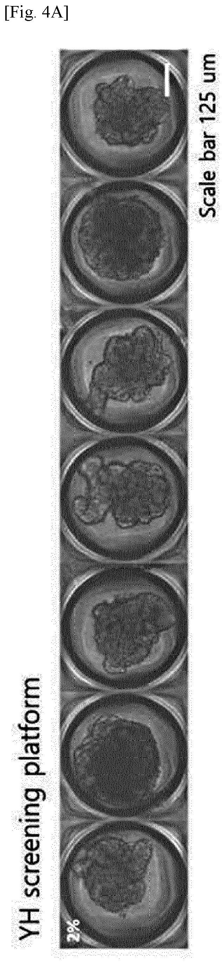

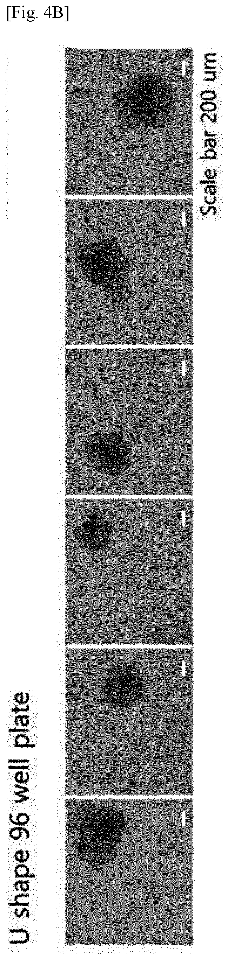

Moreover, the results are illustrated in FIG 4. FIG 4 is a view illustrating the high-speed mass imaging results of Example 1-1 and Comparative Example 1 ((A) Example 1-1, (B) Comparative Example 1).

Referring to FIG 4, it was confirmed that in the case of Example 1-1, the diameters of the cells cultured on the cell culture plate of the present invention were almost uniform. Specifically, when cells were seeded in each sub well at 100 cells/well on average, a uniform organoid that could be comparatively analyzed could be manufactured. In this case, the error range for the organoid size was around 20 µm. Through this, it can be seen that a standard organoid can be produced using the organoid culture method according to the present invention.

In contrast, it could be confirmed that in the case of Comparative Example 1 using a well plate in the related art, cell spheres were formed to have different sizes. The error range of the size of organoids generated due to the growth of multiple cells in the dome morphology of one Matrigel appeared with a deviation of 150 µm or more, and the cells were grown while overlapping, such that it was impossible to perform uniform high-speed mass imaging and experiments.

The base and well plate of the present invention include a convex part and a concave part to fix each other, respectively, and the convex part and the concave part may be connected to each other for the base to firmly fix the well plate, showing that an image in the well plate can be uniformly captured.

In contrast, it can be seen that the size and shape of the organoids cultured in Comparative Example 1 are not uniform. This seems to make an analysis of images difficult because the focal deviation of the imaging is increased in the absence of a plate base.

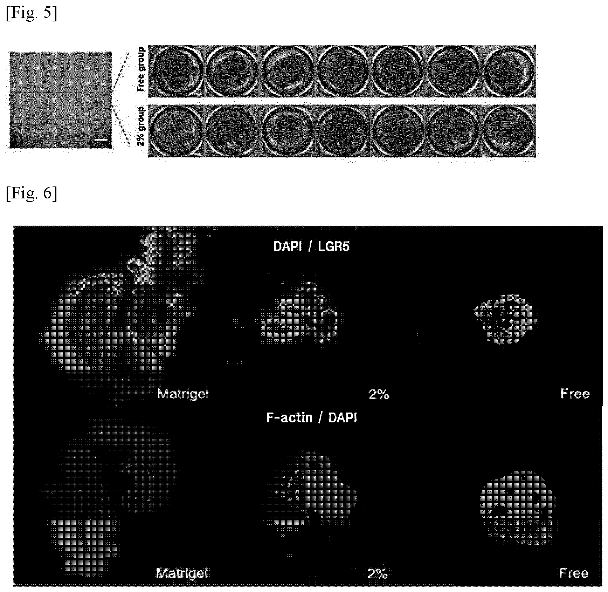

Further, referring to FIG 5, it was confirmed that when the cell culture plate of the present invention was used even without containing Matrigel, organoids were formed well.

FIG 6 confirms the expression level by staining LGR5, which is the most important marker for the formation of colorectal cancer organoids in order to confirm whether the cultured organoids were successfully formed. In addition, the presence of a colon-specific structure in the colorectal cancer organoids of a low-concentration Matrigel group or a group in which Matrigel was not used, formed through F-actin staining, was confirmed.

### Experimental Example 2. Manufacture of standard-type organoid

The cells cultured in Example 1-1 and Comparative Example 1 were subjected to high-speed mass imaging.

The organoids produced in Example 1-1 and Comparative Example 1 were subjected to imaging by an automated plate device, and in this case, the device was allowed to perform imaging by automatically focusing. Image size analysis was performed using a macro program of the ImageJ program.

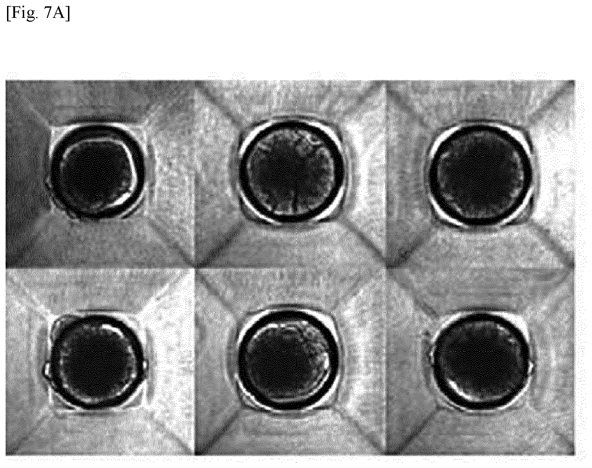

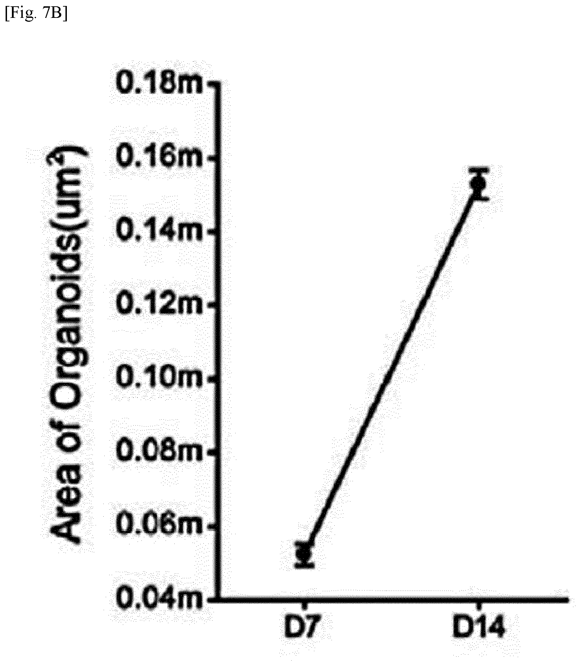

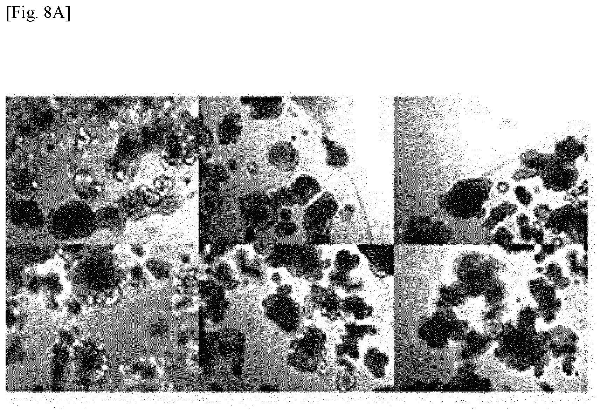

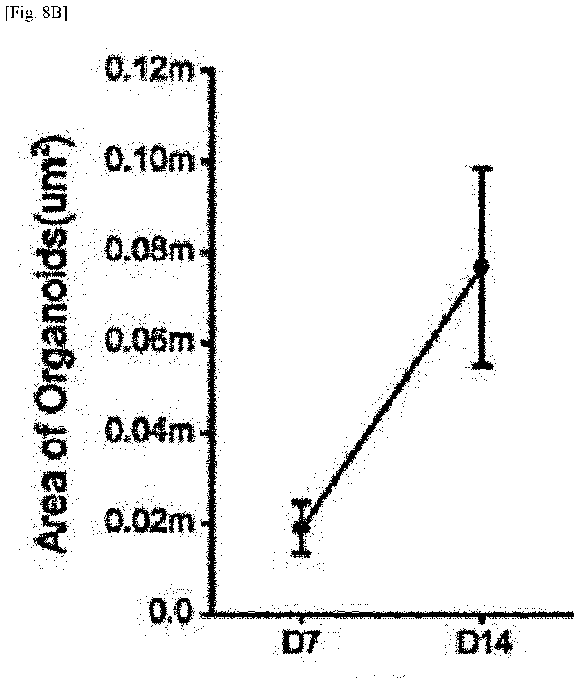

Moreover, the results are illustrated in FIGS. 7 and 8.

FIG 7A is a set of photographs illustrating the high-speed mass imaging results of Example 1-1, FIG 7B is a graph illustrating the area of the organoids cultured for a certain period of time in Example 1, FIG 8A is a set of photographs illustrating the high-speed mass imaging results of Comparative Example 1, and FIG 7B is a graph illustrating the area of the organoids cultured for a certain period of time in Comparative Example 1.

Referring to FIG 7, it can be confirmed that when the organoid prepared in Example 1-1 was subjected to automatic imaging, imaging can be performed without a large error because an imaging height is uniform, and due to this fact, an error range is very small when the actual area is measured.

In particular, when the organoid is cultured using the cell culture plate of the present invention, the organoid is cultured in a uniform size. That is, standardization is possible. As a result of standardization, the focus was automatically determined during image measurement, and a deviation for the measured height was minimized by a connector structure. Accordingly, when the screening image is measured, the deviation is around 20 µm, which is shown to be very small.

Referring to FIG 8, it could be confirmed that in the case of Comparative Example 1, the organoids grew while overlapping each other, and it could be seen that the size and distribution position of the organoids were different, and thus could not be standardized. Therefore, during the measurement of the screening image, an error range was up to 150 µm was exhibited, which is large.

The results as described above are because when organoids are cultured by methods in the related art, since the organoids are grown randomly in Matrigel, it is difficult to uniformly culture a desired organoid, and a measured height is also variable, and thus, there is a limit in which it is difficult to apply the measured height to organoid screening imaging. Therefore, when the area of the organoid cultured for a certain period of time is analyzed, the deviation appears to be very large.

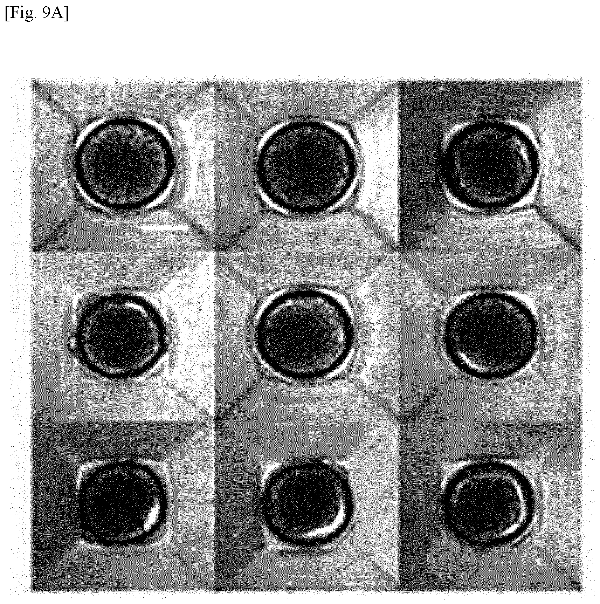

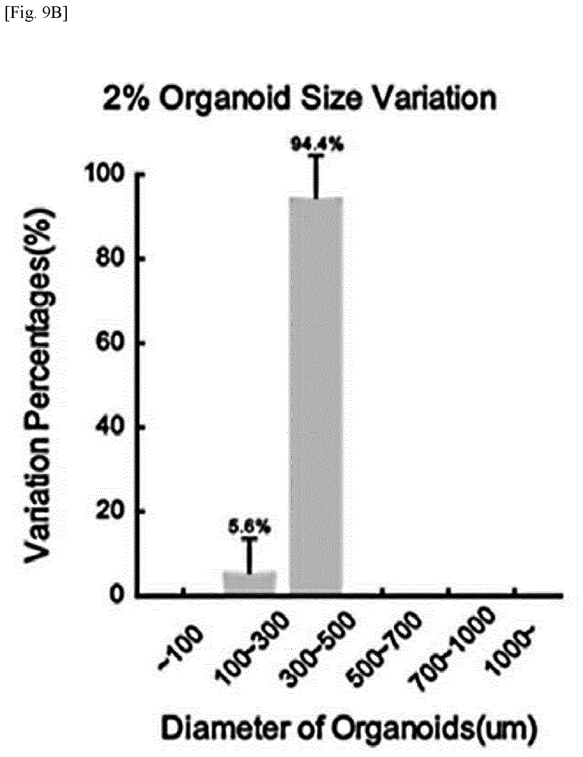

In the case of FIG 9, in the standard-type organoids produced as a whole, the diameter of each organoid was measured using the ImageJ program. A total of 864 wells were subjected to high-speed mass imaging, it was confirmed that a uniform diameter was obtained by analyzing each of the images by ImageJ.

FIG 9 illustrates the imaging results and the size of the organoid when the colorectal cancer cells of Example 1-1 were cultured for 14 days. It can be seen that it is possible to manufacture a standard organoid because the size of the organoids is uniform at 300 to 50 µm.

FIG 10 is a set of image photographs illustrating the organoids according to Examples 1-1 and 1-2 over time (left) and a set of organoid viabilities according to Example 1-1 (right). In the case of FIG 10, it was confirmed how much the cultured organoids were actually maintained by Live/Dead staining of the cultured organoids. First, the cultured standard-type organoid was washed with PBS, and then incubated with Accutase for about 10 minutes.

And then, the standard-type organoid was fragmented into single cells, and after Live/Dead reagents Calcein and EtdH-1 were stored in an incubator for about 30 to 60 minutes, how much the reagents were each present by placing the reagents in a C-Chip was confirmed under a fluorescence microscope, and the results thereof are illustrated in FIG 10..

Through FIG 10, it can be seen that organoid formation occurs very well in the absence of Matrigel, and that organoid viability is very high when colorectal cancer cells are cultured for 14 days.

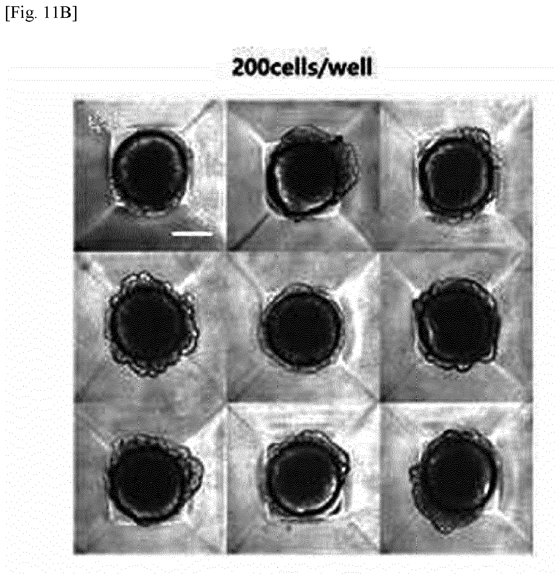

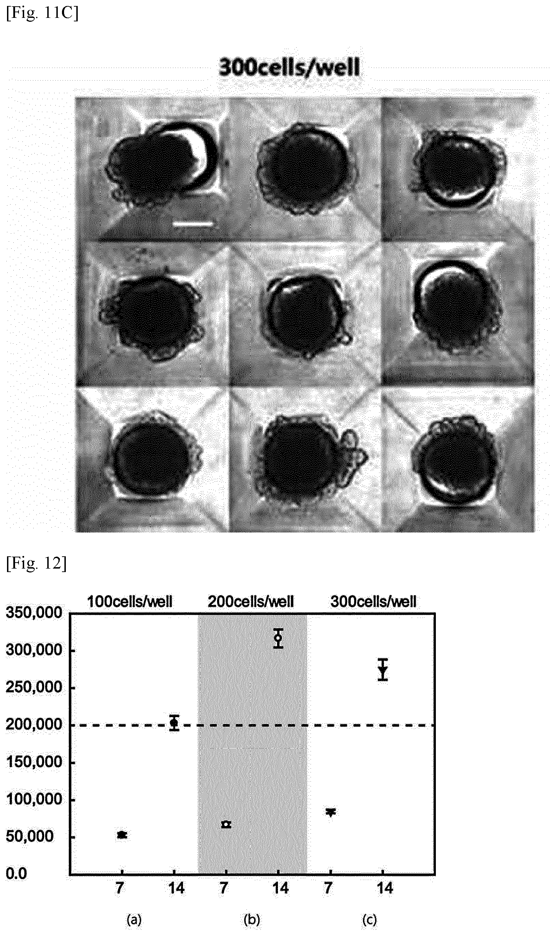

FIG 11 is a set of photographs illustrating the results of high-speed mass imaging of Examples 1-1, 2, and 3, and FIG 9 illustrates the imaging results of colon cancer cells cultured for 14 days in Examples 1-1, 2 and 3 ((A) Example 1-1, (B) Example 2, and (C) Example 3).

Referring to FIG 11A, when cells were seeded in each sub well at 100 cells/well on average, a uniform organoid that could be analyzed and compared could be manufactured (error range: around 20 µm).

In contrast, referring to FIGS. 11B and 11C, it can be confirmed that when cells are seeded in sub wells at more than 100 cells/well, organoids overflow from the sub wells, and thus non-uniform organoids are produced.

FIG 12 is a graph illustrating the results of high-speed mass imaging of Examples 1-1, 2, and 3, and FIG 12 illustrates the results of colon cells cultured for 7 days or 14 days in Examples 1-1, 2, and 3 ((A) Example 1-1, (B) Example 2, and (C) Example 3).

Referring to FIG 12, it shows that the most desirable organoids can be manufactured when cells are cultured at 100 cells/well on average for 14 days. That is, it is considered that an optimum organoid can be differentiated and grown when cells are cultured at 100 cells/well or less on average are cultured for 14 days. In contrast, it could be confirmed that the organoid performance deteriorated when the number of cells seeded in the sub well was increased and the number of culture days was reduced.

For reference, the dotted line in FIG 12 means the maximum space of the sub well of the cell culture plate of the present invention, and means a space in which cells can be cultured. This is considered to be capable of culturing cells at 100 cells/well or less on average.

Although a specific part of the present invention has been described in detail, it will be obvious to those skilled in the art that such a specific description is just a preferred embodiment and the scope of the present invention is not limited thereby. Accordingly, the substantial scope of the present invention will be defined by the appended claims and equivalents thereof.

### [Description of Reference Numerals and Symbols]

100: Well plate
101: Step
110: Main well
120: Sub well
121: Recessed part
130: Space part
140: Concave part
200: Connector for large-capacity and high-speed HCS
210: Base
220: Cover
240: Convex part

## Claims

1. A method of producing an organoid, the method comprising:
forming an organoid by culturing cells in a 3D cell culture plate,
wherein in the forming of the organoid, the cell culture plate comprises 0 to 2 vol% of an extracellular matrix-based hydrogel, and
wherein the 3D cell culture plate comprises:
a well plate comprising a plurality of main wells and a plurality of sub wells formed at lower portions of the main wells to be injected with a cell culture solution and comprising recessed parts on a bottom surface thereof; and a connector for large-capacity and high-speed high content screening (HCS), which supports the well plate, and
the connector for high content screening (HCS) comprises a base equipped with a fixing means so as to be attached to and detached from a lower end of the well plate and a cover positioned on an upper portion of the well plate to be coupled to the base,
the main well has a step formed so as to be tapered at a predetermined site, and the step has an inclination angle (θ) ranging from 10 to 60° with respect to a wall of the main well.

2. The method of claim 1, wherein the extracellular matrix-based hydrogel is Matrigel.

3. The method of claim 1, wherein the cells are normal cells or cancer cells, and
the cell culture period is 1 to 14 days.

4. The method of claim 1, wherein a size of the organoids is 300 to 500 µm in diameter.

5. The method of claim 1, wherein the sub well has an inclined surface formed so as to taper toward a recessed part.

6. The method of claim 1, wherein the sub well has an inclined surface formed so as to taper toward the recessed part,
the sub wells have an upper end diameter ranging from 3.0 to 4.5 mm,
the recessed parts have an upper end diameter ranging from 0.45 to 1.5 mm,
an inclined surface (θ₂) between the sub well and the recessed part ranges from 40 to 50°, and
a length ratio of the diameter of the sub wells to the diameter of the recessed parts ranges from 1:0.1 to 0.5.

7. The method of claim 1, wherein the main well has an individual volume ranging from 100 to 300 µl,
the recessed part has an individual volume ranging from 20 to 50 µl, and an individual volume ratio of the main well to the recessed part is 1 : 0.1 to 0.5 on average.

8. The method of claim 1, wherein the main well comprises a space part between the step and the sub well,
the space part has a height (aₕ) ranging from 2.0 to 3.0 mm on average,
the sub well has a height (bₕ) ranging from 1.0 to 2.0 mm on average, and
a height ratio (aₕ:bₕ) of the space part to the sub well ranges from 1:0.3 to 1.

9. The method of claim 1, wherein the cells are seeded in the sub wells of the cell culture plate at 100 to 300 cells/well.

10. An organoid having a diameter of 300 to 500 um, produced according to any one of claims 1 to 9.
